Europäisches Patentamt

European Patent Office

Office européen des brevets

(11) Publication number: **0 055 041**
**B2**

(12) NEW EUROPEAN PATENT SPECIFICATION

(45) Date of publication of the new patent
specification: **24.10.90**

(21) Application number: **81305708.0**

(22) Date of filing: **03.12.81**

(51) Int. Cl.⁵: **A 61 K 45/08,** A 61 K 9/72 //
(A61K45/08, 31:40, 31:43,
31:44, 31:545, 31:60, 31:635,
31:65, 31:73)

(54) Composition for curing respiratory diseases.

(30) Priority: **04.12.80 JP 170222/80**

(43) Date of publication of application:
**30.06.82 Bulletin 82/26**

(45) Publication of the grant of the patent:
**22.01.86 Bulletin 86/04**

(45) Mention of the opposition decision:
**24.10.90 Bulletin 90/43**

(84) Designated Contracting States:
**CH DE FR GB IT LI**

(56) References cited:
GB-A-2 008 945
US-A-3 594 476
US-A-3 715 432

CHEMICAL ABSTRACTS, vol. 91, no. 25, 17th
December 1979, page 217, no. 206019a,
Columbus Ohio (USA); R.J. KING et al.:
"Physicochemical properties of dipalmitoyl
phosphatidylcholine after interaction with an
apolipoprotein of pulmonary surfactant"

(73) Proprietor: **BYK GULDEN LOMBERG
CHEMISCHE FABRIK GMBH
Postfach 6500 Byk-Gulden-Strasse 2
D-7750 Konstanz (DE)**

(72) Inventor: **Yoshida, Tsunemasa
1823-28, Shimoyugi Hachioji-shi
Tokyo 192-03 (JP)**
Inventor: **Wakabayashi, Toshio
30-30-2, Nagayama 5-chome Tama-shi
Tokyo 206 (JP)**
Inventor: **Matsumoto, Keizo
672-1-310, Takao-machi
Nagasaki-shi Nagasaki-ken 852 (JP)**

(56) References cited:
**CHEMICAL ABSTRACTS, vol. 89, no. 23, 4th
December 1978, page 146, no. 191966q,
Columbus Ohio (USA); L. PERELES et al.:
"Effects of soybean lecithin on the morphology
and surface of mouse lung"**

**Aerosole, Möglichkeiten und Probleme einer
Darreichungsform, Dr. rer. nat. Karl Thoma,
Werbe- und Vertriebsgesellschaft Deutscher
Apotheker m.b.H., D-6000 Frankfurt/Main, 1979
K. Takada et al., Chem. Pharm. Bull. 28(9),
2806-2808 (1980)**

EP 0 055 041 B2

Courier Press, Leamington Spa, England.

## Description

Background of the Invention

The present invention relates to a composition, which contains a lung surfactant, for curing respiratory diseases.

As drugs for curing respiratory diseases, there are such drugs as antibiotics and chemotherapeutics as well as antitussives, expectorants, bronchodilators, antiallergic drugs, antiinflammatory drugs, and anti-cancer drugs. These medicines for curing respiratory diseases are used by general administration such as oral administration, intravenous injection and intramuscular injection; however, in the case of some therapeutic medicines, especially antibiotics, chemotherapeutics and antiallergic drugs like antiasthmatics, the administration by means of inhalation or infusion is also adopted.

The administration of a drug by inhalation or infusion is what is called a local treatment effected by a direct application of the drug to the affected part and can be expected to produce less side effects as compared with the general administration of a drug mentioned above. However, the application of a drug by inhalation or infusion to the respiratory apparatus inclusive of naris, throat, trachea and lung sometimes results in insufficient absorption of the drug through the mucous membrane depending upon the drug and thus it is at a disadvantage in being unable to achieve enough indirect remedial effect attributable to an increase of the concentration of the drug in the blood. It is impracticable to administer some drugs by inhalation or infusion as they irritate the mucous membrane, for instance of the respiratory tracts of the bronchi, causing coughing.

On the other hand, lung surfactant protects the respiratory epithepia by covering the inner walls of the alveoli of lung and also has a physiological function which is important for animals to maintain their respiratory function properly. More particularly, lung surfactant has a singular surface activity which makes the surface tension of the inner surface of the alveolium vary in accordance with expiration and inspiration and contributes to the maintenance of interalveolium functional stabilization to exercise an anti-atelectatic action. It is known that this surfactant contains phospholipids, neutral lipids and protein as ingredients and has dipalmitoyl lecithin as a main ingredient. T. Fujiwara et al. recently reported that they obtained an artificially prepared lung surfactant having a higher surface activity by adding dipalmitoyl lecithin to a surfactant recovered from bovine lung and that they achieved good results for the therapy of idiopathic respiratory distress syndrome (IRDS) by instillation of said lung surfactant dispersion to the respiratory tracts of premature babies (Pediatric Clinics, Vol. 32, No. 7, p. 1335, 1979).

In US—A—3 594 476 and US—A—3 715 432 there are disclosed compositions containing DL dipalmitoyl-α-lecithin. However, this compound is not a lung surfactant and therefore these compositions do not have the high surface activity required in the present invention.

In Chemical Abstracts, Volume 91, No. 206019a (1979), there are disclosed the physicochemical properties of dipalmitoyl phosphatidylcholine after interaction with an apolipoprotein from a lung surfactant. This disclosure does not relate to compositions for inhalation or infusion for curing respiratory diseases.

The inventors of this invention have discovered, in the course of strenuous research work to find out how to increase the absorption of drugs through the mucous membranes of the respiratory apparatus, how to improve the dispersibility of drugs to the peripheral airways and alveoli, or how to decrease irritation of the mucous membranes by drugs, that the combined use of such drugs with lung surfactant enhances absorption and reduces irritation of the membranes.

Therefore, according to the present invention, there is provided a composition for inhalation or infusion for curing respiratory disease which contains a medicine for respiratory disease and a lung surfactant in a ratio of at least 0.01 part by weight of lung surfactant per 1 part by weight of the medicine for respiratory disease, wherein the lung surfactant comprises a natural lung surfactant obtained from animal lung and having a minimum surface tension of 20 dyne/cm or less when the surface activity is measured on a modified Wilhelmy balance and a stability index of 1.0 or more, which will be referred to later.

Brief Description of the Drawing

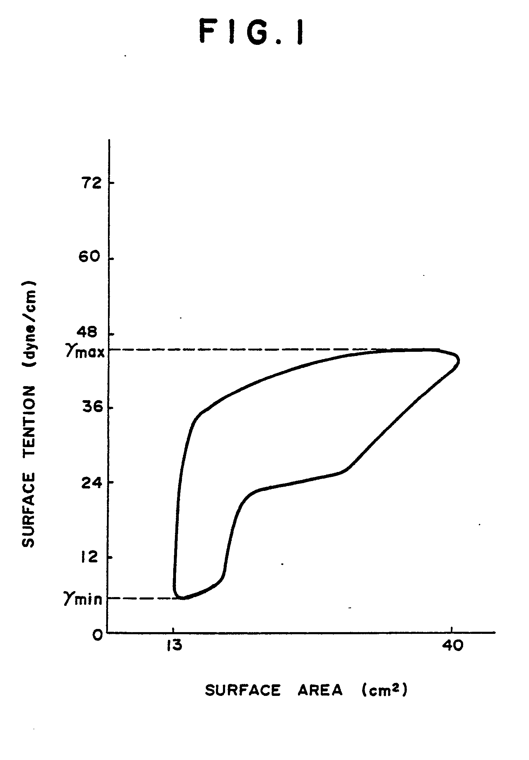

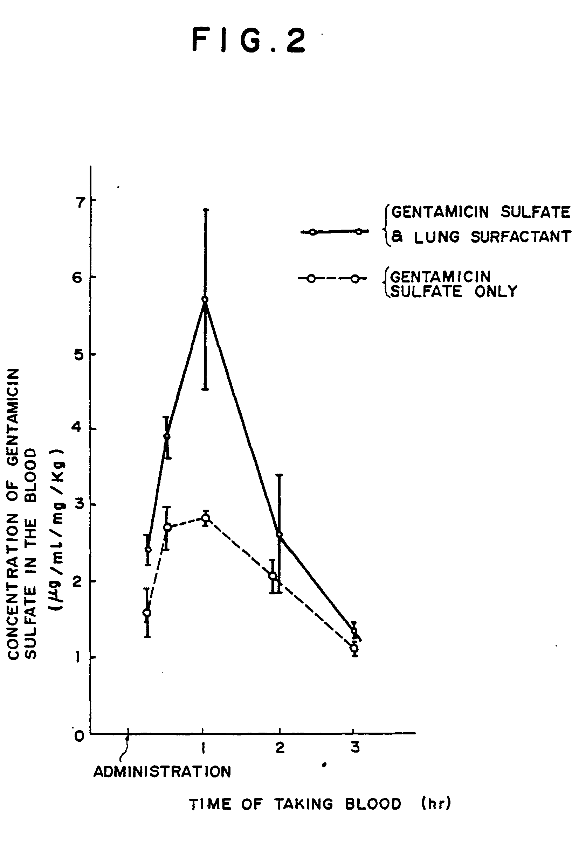

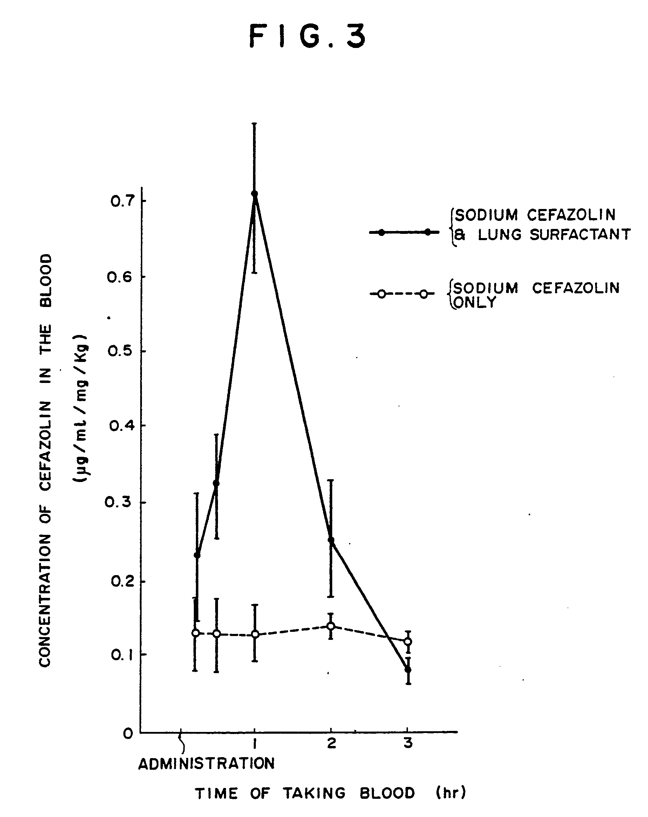

Fig. 1 shows a diagram of surface tension of lung surfactant versus surface area. Fig. 2 shows the change in concentration of gentamicin sulfate in the blood with time after the administration of a medicine for respiratory disease (gentamicin sulfate plus lung surfactant) of the present invention to rabbits. Fig. 3 shows the change in concentration of sodium cefazolin in the blood with time after the administration of a second medicine for respiratory disease (sodium cefazolin plus lung surfactant) of the present invention to rabbits.

Description of the Preferred Embodiment

As the medicine for respiratory disease to be used in the present invention, there are drugs such as chemotherapeutics, antibiotics, anticancer drugs, antiasthmatics, antiallergics, bronchodilator and antitussives.

Accordingly, any of the medicines for respiratory disease can be used so far as they can be expected to be of medical efficacy when they are directly administered by inhalation or infusion to, for instance, the naris, throat, bronchial tube, respiratory tract and alveolium; however, it is desirable to use such ones that will not substantially react with the lung surfactant or that will not reciprocally or unilaterally degrade the pharmaceutical activity or physiological activity when they coexist in a mixture. As concrete examples of desirable medicines, chemotherapeutics include sulfonamides having an isoxazole nucleus, such as sulfamethoxazole, sulfonamides having a pyrimidine nucleus, such as sulfadimethoxine, sulfonamides having a pyrazole nucleus, such as sulfaphenazole, and antituberculotics such as PAS and INH.

As antibiotics, there are antibiotics of penicillin type, such as amoxicillin, ampicillin, ciclacillin, carbenicillin, talanpicillin and benzylpenicillin, antibiotics of cephalosporin type, such as cephalexin, cephaloglycin, cefradine, cefazolin and cephalothin, antibiotics of aminoglucoside type, such as gentamicin sulfate and amikacin sulfate, antibiotics of macrolide type, such as erythromycin and oleandmycin, antibiotics of tetracycine type, such as minocycline and tetracycline, and mitomycin C having a high antitumor activity. Also, anti-cancer drugs aimed at curing lung cancer, antiinflammatory drugs, antiallergic drugs, or immunoglobulins are desirable medicines.

The lung surfactant to be used in the present invention is a natural one. Natural lung surfactant can be obtained by extraction from the washings or tissues of animal lung, such as bovine lung and pig lung; however, since such surfactant has a possible hazard of containing lipoproteins originating from the respective animals, it is desirable to use a lung surfactant which has had its proteins removed, for instance by following the Folch procedure.

The constitution of the composition for curing respiratory diseases according to the present invention is 0.01 part by weight or more, preferably 0.1 to 100 parts by weight, of the lung surfactant against 1 part by weight of the drug. Besides the drug, the composition may contain a proper amount of for instance an excipient or a diluent.

Since the composition for curing respiratory diseases of the present invention is administered to the diseased part by inhalation or infusion, it is preferable to prepare it in the form of a fine powder or a liquid dispersion in water or a suitable medium (ethanol, for instance). As the method for preparing a uniform liquid dispersion, there is one in which the medicine is firstly dissolved in distilled water or a saline solution when the medicine is water-soluble, or is thoroughly dispersed and suspended, in for instance water, by means, for instance of ultrasonic waves to make a liquid dispersion having particles of less than a few microns when the medicine is not water-soluble, and next a desired amount of a lung surfactant is added to the thus prepared solution or liquid dispersion. A homogeneous solution or liquid dispersion is obtained by means of, for instance, an ultrasonic wave. A similar homogeneous liquid dispersion can be prepared by adding a suitable amount of distilled water of a saline solution to a mixture of a drug and lung surfactant with the use of, for instance an ultrasonic wave. The thus obtained liquid dispersion is administered by inhalation with use of, for instance a nebulizer or by infusion with use of, for instance a catheter. For preservation's sake, the liquid dispersion can be lyophilized once and again dispersed in, for instance distilled water at the time of use to be administered by inhalation or infusion. In order to prepare a fine uniform medical powder, a medicine and lung surfactant are mixed thoroughly with a medically permissible suitable fine powdered excipient, or the medicine and dry lung surfactant are mixed thoroughly. A fine medical powder can be administered with the use of a suitable powder inhalator.

The lung surfactant which is used in the composition for curing respiratory diseases of the present invention is not only functional in promoting the absorption of the medicine for curing respiratory diseases through the respiratory membranes and increasing the concentration of the medicine in the blood but is also functional in assisting the proper respiratory function of the lung. For instance, it is known that atelectasis is experienced in, for instance bacterial pneumonia, which is said to result from the degradation of lung surface activity. The administration of a medicine of the present invention consisting of an antibiotic or chemotherapeutic and lung surfactant in such a case not only produces the effect of curing the infection with an increased concentration of the antibiotic or chemotherapeutic in the blood, but also supplies the highly active lung surfactant to make up for the decreased surface activity, thus meeting expectations of quick improvement of the disease.

The present invention is illustrated below with reference to the examples. The surface activity of the lung surfactant was determined according to the method mentioned below.

Determination of Surface Activity
of Lung Surfactant and Calculation
of Stability Index

Lung surfactant was dispersed in distilled water in concentration of 1% by weight and was treated with ultrasonic waves to obtain a uniform liquid dispersion, which was then used as a test sample. A Whilhelmy balance of Acoma make was used in the measurement. 5 µl of said test sample was gently layered over 50 ml of saline solution in the Teflon* trough of the balance. The surface film area was compressed cyclically in the range of a maximum of 40 cm² to a minimum of 13 cm² at the rate 0.3 cycle/min to record a surface tension area diagram (hysteresis loop) by use of an X—Y recorder. The minimum surface tension (γ min) value (the bottom of the loop) and the maximum surface tension (γ max) value (the top of the loop) were obtained from the hysteresis loop. The hysteresis loop became substantially invariant in shape after the 5th or 6th cycle from the start (see Fig. 1). This loop was used to determine γ min and γ max. The stability index (S.I.) was obtained from the following equation:

* 'Teflon' is a Registered Trade Mark.

$$S.I. = \frac{2(\gamma \max - \gamma \min)}{\gamma \max + \gamma \min}$$

## Example 1

3 kg of bovine lungs were homogenized with a saline solution, had its cellular debris removed, and was subjected to density gradient centrifugation to obtain crude lung surfactant. The thus obtained surfactant was dissolved in a solvent consisting of chloroform and methanol in a volume ratio of 2:1, from which undissolved substances were removed. The mixed solution was contacted with 0.5% saline solution by weight to remove protein (Folch procedure). Thereafter, the organic layer was collected and the solvents were removed to obtain about 3 g of white purified lung surfactant.

Its minimum surface tension ($\gamma$ min) was 5 dyne/cm and its S.I. was 1.59.

800 mg of gentamicin sulfate was dissolved in 20 ml of water, to which 800 mg of the above-mentioned lung surfactant was added and the mixture was treated with ultrasonic waves to give a homogeneous liquid dispersion.

Then, an experiment was conducted using a group consisting of three (Japanese white) rabbits weighing approximately 3 kg. The anterior jugulum of each rabbit was cut open to expose the trachea, which was then punctured with an 18-gauge Teflon* sheath needle (70 cm long). The needle was further led through to the bronchus to infuse 2 ml of the liquid dispersion containing 80 mg of gentamicin sulfate and 80 mg of lung surfactant per rabbit. Blood samples were taken at regular intervals to measure the concentration of gentamicin sulfate in the serum. A control experiment was also conducted with a group of three similar rabbits. 2 ml of water containing 80 mg of gentamicin sulfate dissolved therein was infused to each rabbit according to the same method mentioned above and the concentration of gentamicin sulfate was measured.

The results of these experiments are shown in Fig. 2. In Fig. 2 and Fig. 3, the concentration of the medicine is shown by the absorption index ($\mu$g/ml/mg/kg) adjusted by the amount of the medicine administered per rabbit weight.

As clearly shown in Fig. 2, the concentrtion of the medicine in the blood is very high in the case where gentamicin sulfate is used in combination with lung surfactant as compared with the case where gentamicin sulfate is used alone.

When the observation was made as to the state of the rabbits treated with the medicine, it was inferred that the administration of gentamicin sulfate and lung surfactant together gave less stimulus to the membranes of the bronchi than the treatment with gentamicin sulfate alone from the fact that the former caused the rabbits to cough less often than the latter.

## Example 2

5.0 g of sodium cefazolin and 500 mg of lung surfactant obtained in Example 1 were added to 20 ml of water and were treated with ultrasonic waves to obtain a homogeneous liquid dispersion.

An experiment was conducted in the same way as in Example 1, wherein a group consisting of three rabbits was used and each of them had an infusion of 2 ml of a dispersion liquid containing 500 mg of sodium cefazolin and 50 mg of lung surfactant. Blood was taken at regular intervals to measure the concentration of sodium cefazolin therein. A control experiment was conducted with a group consisting of three rabbits. Each of the rabbits was infused with 2 ml of an aqueous solution containing 500 mg of sodium cefazolin to measure its concentration in the blood. The results are shown in Fig. 3.

As is clearly shown in Fig. 3, when sodium cefazolin is used together with lung surfactant, the concentration of sodium cefazolin in the blood is two to five times as high when compared with its concentration when used alone. The membranes of the bronchi had less stimulus when sodium cefazolin was used together with lung surfactant.

## Example 3

50 mg of mitomycin C and 200 mg lung surfactant obtained in Example 1 were added to 25 ml of water and were treated with ultrasonic waves to obtain a homogeneous liquid dispersion.

An experiment was conducted in the same way as in Example 1, wherein a group consisting of seven rabbits was used and each of them had an infusion of 3 ml of a liquid dispersion containing 6 mg of mitomycin C and 24 mg of lung surfactant. Blood was taken after 30 minutes to measure the concentration of mitomycin C therein. A control experiment was conducted with a group consisting of seven rabbits. Each of the rabbits was infused with 3 ml of an aqueous solution containing 6 mg of mitomycin C to measure its concentration in the blood.

When mitomycin C was used together with lung surfactant, the concentration of mitomycin C in the blood was 1.18 ± 1.0 $\mu$g/ml, and on the other hand when mitomycin C was used alone, its concentration was 0.10 ± 0.06 $\mu$g/ml.

## Claims

1. A composition for inhalation or infusion for curing respiratory disease which contains a medicine for respiratory disease and a lung surfactant in a ratio of at least 0.01 part by weight of lung surfactant per 1 part by weight of the medicine for respiratory disease, wherein the lung surfactant comprises a natural lung surfactant obtained from animal lung and having a minimum surface tension of 20 dyne/cm or less when the surface activity is measured on a modified Wilhelmy balance and a stability index of 1.0 or more.

2. A composition according to claim 1, wherein the medicine for respiratory disease is an antibiotic or chemotherapeutic.

3. A composition according to claim 1, wherein the medicine for respiratory disease is an anti-cancer drug.

4. A composition according to claim 1, wherein the medicine for respiratory disease is an antiallergic drug.

5. A composition according to claim 1, wherein the medicine for respiratory disease is an antiinflammatory drug.

6. A composition according to any of claims 1 to 5, in the form of a dispersion in water or an appropriate dispersing medium.

**Patentansprüche**

1. Zubereitung für eine Inhalation oder Infusion zur Heilung von Erkrankungen der Atmungsorgane, die einen Wirkstoff gegen Erkrankung der Atmungsorgane und eine lungenoberflächenaktives Mittel in einer Menge von wenigstens 0,01 Gew.-Teilen des lungenaktiven Mittels pro 1 Gew.-Teil des Wirkstoffs gegen die Erkrankung der Atemorgane enthält, wobei das lungenoberflächenaktive Mittel ein aus der Lunge eines Lebewesens erhältliches lungenoberflächenaktives Mittel ist, das bei Messung mit einer modifizierten Wilhelmy-Waage eine minimale Oberflächenspannung von 20 dyn/cm oder weniger hat und einen Stabilitätsindex von 1,0 oder mehr aufweist.

2. Zubereitung nach Anspruch 1, dadurch gekennzeichnet, daß der Wirkstoff gegen die Erkrankung der Atemorgane ein Antibiotikum oder Chemotherapeutikum ist.

3. Zubereitung nach Anspruch 1, dadurch gekennzeichnet, daß der Wirkstoff gegen die Erkrankung der Atemorgane ein Antikrebswirkstoff ist.

4. Zubereitung nach Anspruch 1, dadurch gekennzeichnet, daß der Wirkstoff gegen die Erkrankung der Atemorgane ein antiallergischer Wirkstoff ist.

5. Zubereitung nach Anspruch 1, dadurch gekennzeichnet, daß der Wirkstoff gegen die Erkrankung der Atemorgane ein antiinflammatorischer Wirkstoff ist.

6. Zubereitung nach einem der Ansprüche 1 bis 5 in Form einer Dispersion in Wasser oder einem geeigneten Dispergierungsmedium.

**Revendications**

1. Composition pour inhalation ou infusion pour la guérison des maladies respiratories qui contient un remède contre les maladies respiratoires et un surfactant pulmonaire en proportion d'au moins 0,01 partie en poids de surfactant pulmonaire par partie en poids du remède contre les maladies respiratoires, dans laquelle le surfactant pulmonaire contient un surfactant pulmonaire naturel obtenu à partir de poumons d'animaux et ayant une tension supericielle minimale de 20 dynes/cm ou moins, ladie activité superficielle étant mesurée sur une balance WILHELMY modifiée et ayant l'index de stabilité de 1,0 ou plus.

2. Composition selon la revendication 1, dans laquelle le remède contre les maladies respiratoires est un antibiotique ou une substance pour la chimiothérapie.

3. Composition selon la revendication 1, dans laquelle le remède contre les maladies respiratoires est un médicament anticancéreux.

4. Composition selon la revendication 1, dans laquelle le remède contre les maladies respiratoires est un médicament anti-allergique.

5. Composition selon la revendication 1, dans laquelle le remède contre les maladies respiratoires est un médicament anti-inflammatoire.

6. Composition selon l'une quelconque des revendications 1 à 5, sous forme d'une dispersion dans l'eau ou dans un milieu dispersant approprié.

# FIG. 1

# F I G . 2

Legend:
- ○——○ { GENTAMICIN SULFATE & LUNG SURFACTANT
- ○---○ { GENTAMICIN SULFATE ONLY

Y-axis: CONCENTRATION OF GENTAMICIN SULFATE IN THE BLOOD ( $\mu g$ / ml / mg / Kg )

X-axis: TIME OF TAKING BLOOD (hr)

ADMINISTRATION

# F I G . 3